# EUROPEAN PATENT APPLICATION

(11) **EP 4 295 814 A1**
(43) Date of publication of application: **27.12.2023**
(21) Application number: 22756433.3
(22) Date of filing: 11.02.2022
(51) Int. Cl.: A61F 2/12, A61F 2/00, A61L 27/18, A61L 27/34

(54) **ARTIFICIAL IMPLANT**

(30) Priority: 19.02.2021 KR 20210022536
(71) Applicant: Osstemimplant Co., Ltd., Gangseo-gu Seoul 07789 (KR)
(72) Inventor: SIM, Eun Jung, Seoul 07568 (KR); KIM, Byung Hwi, Yongin-si, Gyeonggi-do 16968 (KR); KIM, Min Kyoung, Incheon 22765 (KR); SONG, Ju Dong, Busan 48272 (KR); JANG, Il Seok, Yangsan-si, Gyeongsangnam-do 50653 (KR)
(74) Representative: Beck & Rössig European Patent Attorneys
(86) International application number: PCT/KR2022/002068
(87) International publication number: WO 2022/177234

(57) **Abstract**

Disclosed is an artificial implant comprising: a silicone shell; and a filler filling the interior of the shell, wherein at least a portion of the shell is a rupture-prevention part comprising two or more silicone layers and one or more reinforcing material layers interposed therebetween.

## Description

### [Technical Field]

The present invention relates to an artificial implant.

### [Background Art]

Artificial breast implants are Class IV medical devices in which a gel having appropriate viscosity or saline is filled in a silicone shell forming the outer shell of the implant, and require special stability in the living body. In the implant industry, properties required as a medical device have been secured by adjusting the thickness of a silicone shell or changing a filler component, and research and development is being conducted to realize a natural shape by adjusting the properties of a filler.

However, artificial implants still have a potential risk for post-implantation side effects. Particularly, a rippling phenomenon frequently occurs due to folding of a silicone shell. The rippling phenomenon is a side effect in which wrinkles formed on the implant surface are revealed as wavy patterns on the skin surface of the breast. This phenomenon may reduce the aesthetic satisfaction of users, and as folding continuously occurs at a specific site of a silicone shell which is the outer shell of implants, durability is degraded at the corresponding site, and thus implant rupture may be caused.

As described above, when artificial implants rupture, a filler present in the implant may leak out. As the filler leaks out, inflammatory reactions may occur inside the human body, and furthermore, tissue necrosis, bacterial infections, capsular contracture, breast implant-associated anaplastic large cell lymphoma (BIA-ALCL), and the like, which may cause extreme pain to users due to overactive immune responses, may be caused. Particularly, users who develop capsular contracture need to undergo a reoperation, and users who develop BIA-ALCL may face the risk of death.

In order to solve the above-described problems of the conventional artificial implant, it is most important to prevent the rupture of implants in advance. Particularly, since the rippling phenomenon continuously occurs at a specific site of a silicone shell, which is the outer shell of implants, to degrade durability, there is a need for the research and development of an artificial implant capable of imparting mechanical properties to the corresponding site and a method of manufacturing the same.

### [Disclosure]

### [Technical Problem]

The present invention is directed to providing an artificial implant having improved rupture resistance.

### [Technical Solution]

One aspect of the present invention provides an artificial implant, which includes: a silicone shell; and a filler filled in the shell, wherein at least a portion of the shell is a rupture-resistant portion including two or more silicone layers and one or more reinforcing material layers interposed therebetween.

In an embodiment, a portion of the shell, which is not the rupture-resistant portion, may have a thickness of 0.40 to 0.80 mm, and the rupture-resistant portion may have a thickness of 0.40 to 1.20 mm.

In an embodiment, the shell may further include a barrier film layer.

In an embodiment, the rupture-resistant portion may be provided in a side portion.

In an embodiment, the reinforcing material may be at least one selected from polyester, polyurethane, polyvinyl chloride, nylon, polyethylene glycol, polyethylene oxide, polyhydroxyethylmethacrylate, poly(N-isopropylacrylamide), polycaprolactone, polylactic acid, polyglycolic acid, poly[(lactic-co-(glycolic acid))], polydioxanone, poly[(L-lactide)-co-(caprolactone)], poly(ester urethane), poly[(L-lactide)-co-(D-lactide)], poly[ethylene-co-(vinyl alcohol)], polyacrylic acid, polyvinyl alcohol, polyvinylpyrrolidone, gelatin, alginate, carrageenan, chitosan, hydroxyalkyl cellulose, alkyl cellulose, silicone, rubber, polyacrylacetate, titanium, titanium alloys, zirconia, alumina, nichrome, cochrome, and gold.

In an embodiment, the reinforcing material layer may have at least one structure selected from a mesh, a woven fabric, a non-woven fabric, and a sheet.

In an embodiment, the rupture-resistant portion may consist of silicone and a reinforcing material in a weight ratio of 55 to 95:5 to 45.

In an embodiment, the rupture-resistant portion may have a tensile strength of 25 N or more.

In an embodiment, the rupture-resistant portion may have an elongation rate of 300 to 500%.

### [Advantageous Effects]

According to an aspect, the rupture resistance of an artificial implant can be improved.

However, it is to be understood that the effect of an aspect of the specification is not limited to the above-described effect but include all effects deducible from the configuration described in the detailed description of the specification or in the claims.

### [Description of Drawings]

FIG. 1 shows a longitudinal sectional view of an artificial implant according to an embodiment of the specification.
FIG. 2 shows a longitudinal sectional view of an artificial implant according to an embodiment of the specification, in which a rupture-resistant portion is provided in a side portion.
FIG. 3 shows a longitudinal sectional view of an artificial implant according to an embodiment of the specification, in which a side portion has varying thicknesses.
FIG. 4 shows one example of a rupture-resistant portion of an artificial implant according to an embodiment of the specification.
FIG. 5 shows another example of a rupture-resistant portion of an artificial implant according to an embodiment of the specification.
FIG. 6 show a result of evaluating the tensile strength of a sample related to a rupture-resistant portion of an artificial implant according to an embodiment of the specification.
FIG. 7 show a result of evaluating the elongation rate of a sample related to a rupture-resistant portion of an artificial implant according to an embodiment of the specification.

### [Modes of the Invention]

Hereinafter, an aspect of the specification will be described with reference to the accompanying drawings. However, it should be understood that the descriptions of the specification can be implemented in various other forms, and that it is not intended to limit the present invention to the exemplary embodiments. Also, in the drawings, descriptions of parts unrelated to the detailed description are omitted to clearly describe an aspect of the specification. Throughout the specification, like numbers refer to like elements.

Throughout the specification, the phrase a certain part is "connected" to another part means that the certain part is not only "directly connected" to the other part but also "indirectly connected" to the other part through another member interposed between the two parts. Also, the phrase a certain part "includes" a certain element means that the certain part may further include, instead of excluding, another element unless particularly indicated otherwise.

When a numerical value is presented in the specification, the value has the precision of significant digits provided in accordance with the standard rules in chemistry for significant digits unless its specific range is stated otherwise. For example, the numerical value 10 includes the range of 5.0 to 14.9 and the numerical value 10.0 includes the range of 9.50 to 10.49.

Hereinafter, an embodiment of the specification will be described in detail with reference to the accompanying drawings.

### Artificial implant 100

An artificial implant 100 according to an aspect includes: a silicone shell 110; and a filler 120 filled in the shell 110, wherein at least a portion of the shell 110 is a rupture-resistant portion including two or more silicone layers 111, 113, and 115 and one or more reinforcing material layers 112 and 114 interposed therebetween.

The artificial implant may have varying shapes depending on the insertion site or the purpose of the procedure and may be inserted into various body parts such as the breast, nose, buttocks, forehead, and the like, but the shape thereof is not particularly limited.

In a non-limiting example, the artificial implant 100 may serve as an implant in the body by injecting the filler 120 into the silicone shell 110. The filler 120 may be a silicone gel. In an example, the silicone gel may be polyorganosiloxane. The polyorganosiloxane may be one selected from the group consisting of dimethylsiloxane, methyl hydrogen siloxane, methylphenylsiloxane, diphenylsiloxane, dimethylvinylsiloxane, trifluoropropylsiloxane, and a mixture of two or more thereof, but the type thereof is not particularly limited as long as it can realize the functional characteristics of an artificial implant.

When the artificial implant 100 is an artificial breast implant, the cross-section thereof may have one shape selected from a circular shape, an oval shape, and a teardrop shape, but the present invention is not limited thereto. Generally, since users have different breast shapes, the shape of the artificial implant may be selected in consideration of an original shape and preference of users such as a desired shape, a desired size, and the like.

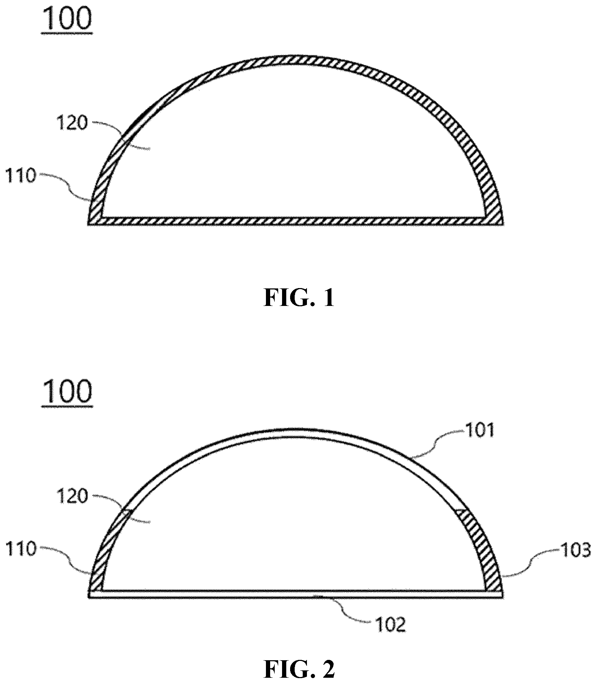

FIGS. 1 to 3 show sectional views of an artificial implant 100 according to an embodiment. Referring to FIG. 1, the artificial implant 100 may include a filler 120 and a silicone shell 110 surrounding the filler 120. The silicone shell 110 may include a rupture-resistant portion in which reinforcing material layers 112 and 114 are interposed between a plurality of silicone layers 111, 113, and 115.

The artificial implant 100 may have an upper portion 101, a lower portion 102, and a side portion 103, and the rupture-resistant portion may be provided in at least a portion of the silicone shell 110, for example, in at least one of the upper portion 101, lower portion 102, and side portion 103 of the implant. FIG. 2 shows an example in which the rupture-resistant portion is provided in only the side portion 103.

The silicone shell 110 may have a thickness of 0.40 to 0.80 mm, for example, 0.40 mm, 0.45 mm, 0.50 mm, 0.55 mm, 0.60 mm, 0.65 mm, 0.70 mm, 0.75 mm, 0.80 mm, or a thickness in a range between two of the above values. In the silicone shell 110, the rupture-resistant portion may have a thickness equal to or relatively thicker than other portions which are not the rupture-resistant portion, and have a thickness of 0.40 to 1.20 mm, for example, 0.40 mm, 0.45 mm, 0.50 mm, 0.55 mm, 0.60 mm, 0.65 mm, 0.70 mm, 0.75 mm, 0.80 mm, 0.85 mm, 0.90 mm, 0.95 mm, 1.00 mm, 1.05 mm, 1.10 mm, 1.15 mm, 1.20 mm, or a thickness in a range between two of the above values. When the silicone shell 110 and the rupture-resistant portion have thicknesses less than 0.40 mm, mechanical properties are insufficient, and thus damage to the artificial implant 100 and aesthetic side effects may easily occur. When the silicone shell 110 has a thickness exceeding 0.80 mm or the rupture-resistant portion has a thickness exceeding 1.20 mm, users who have received the artificial implant 100 may feel discomfort such as a foreign body sensation.

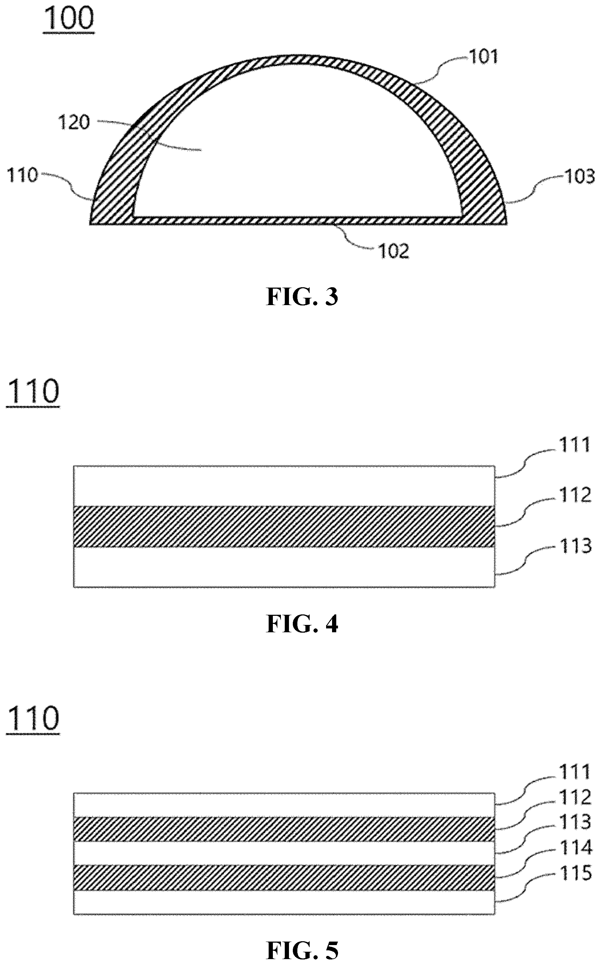

FIG. 3 shows an example of the artificial implant 100 whose thickness is reinforced, and it can be seen that the side portion 103 is thicker than the upper portion 101 and the lower portion 102.

Since the rippling phenomenon of an artificial breast implant generally frequently occurs at the side portion 103 of the implant, the folding and rupture of the artificial implant 100 may be prevented by applying the rupture-resistant portion to only a portion of the implant or varying the thickness of the side portion 103, and degradation of convenience, aesthetics, tactile sensation, and the like of users may also be minimized.

FIGS. 4 and 5 show examples of the rupture-resistant portion. Referring to FIG. 4, it can be seen that a reinforcing material layer 112 is interposed between two silicone layers 111 and 113. Referring to FIG. 5, it can be seen that, even though the total thickness is the same as that in FIG. 4, the thickness of each layer is decreased, and reinforcing material layers 112 and 114 are interposed between three silicone layers 111, 113, and 115, but the rupture-resistant portion is not limited thereto. For example, the silicone layer may consist of multiple layers such as 2, 3, 4, 5, 6, 7, 8, 9, 10, or 10 or more layers, and the reinforcing material layer may consist of multiple layers such as 1, 2, 3, 4, 5, 6, 7, 8, 9 or 9 or more.

The silicone shell 110 may further include a barrier film layer. For example, the silicone shell 110 may consist of a general silicone layer and a barrier film layer. The barrier film layer may prevent a low-molecular-weight silicone component in the filler 120 in the artificial implant 100 from leaking out. In an example, the lowermost layer of the rupture-resistant portion, which is close to the filler 120, may be the barrier film layer, and the barrier film layer, the reinforcing material layer, and the silicone layer may be sequentially laminated.

The reinforcing material may be at least one selected from polyester, polyurethane, polyvinyl chloride, nylon, polyethylene glycol, polyethylene oxide, polyhydroxyethylmethacrylate, poly(N-isopropylacrylamide), polycaprolactone, polylactic acid, polyglycolic acid, poly[(lactic-co-(glycolic acid))], polydioxanone, poly[(L-lactide)-co-(caprolactone)], poly(ester urethane), poly[(L-lactide)-co-(D-lactide)], poly[ethylene-co-(vinyl alcohol)], polyacrylic acid, polyvinyl alcohol, polyvinylpyrrolidone, gelatin, alginate, carrageenan, chitosan, hydroxyalkyl cellulose, alkyl cellulose, silicone, rubber, polyacrylacetate, titanium, titanium alloys, zirconia, alumina, nichrome, cochrome, and gold, but the present invention is not limited thereto. The type of reinforcing material is not limited as long as it is a material that has excellent elasticity and restorability and is harmless to the human body due to having excellent biocompatibility.

The reinforcing material layer may have at least one structure selected from a mesh, a woven fabric, a non-woven fabric, and a sheet. The mesh may be a net knot structure like the holes of a sieve, the woven fabric may be a structure in which fibrous structures are woven by crossing according to a certain rule, the non-woven fabric may be a structure in which fibrous structures are arranged without any certain rule, and the sheet may be a plate-like structure. The structure of the reinforcing material layer may be freely selected according to a material of the reinforcing material. For example, the structure may be a metal mesh such as a titanium mesh or the like, a nylon woven fabric, a polyester non-woven fabric, a rubber sheet, or the like, but the present invention is not limited thereto.

The rupture-resistant portion may consist of silicone and a reinforcing material in a weight ratio of 55 to 95:5 to 45. For example, the weight ratio may be 55:45, 60:40, 65:35, 70:30, 75:25, 80:20, 85:15, 90:10, 95:5, or a weight ratio in a range between two of the above ratios. When the weight ratio is out of the above range, the artificial implant 100 may be excessively cured, or mechanical properties may be degraded.

The rupture-resistant portion may have a tensile strength of 25 N or more. For example, the tensile strength may be 25 N, 26 N, 27 N, 28 N, 29 N, 30 N, 31 N, 32 N, 33 N, 34 N, 35 N, 36 N, 37 N, 38 N, 39 N, 40 N, 41 N, 42 N, 43 N, 44 N, 45 N, 46 N, 47 N, 48 N, 49 N, or 50 N, but the present invention is not limited thereto. Due to the rupture-resistant portion in the silicone shell 110 constituting the outer shell of the artificial implant 100, durability and rupture resistance can be reinforced.

The rupture-resistant portion may have an elongation rate of 300 to 500%, for example, 300%, 310%, 320%, 330%, 340%, 350%, 360%, 370%, 380%, 390%, 400%, 410%, 420%, 430%, 440%, 450%, 460%, 470%, 480%, 490%, 500%, or an elongation rate in a range between two of the above values. When the elongation rate is out of the above range, the user's foreign body sensation resulting from the artificial implant 100 may increase, or desired mechanical properties may not be realized.

In an example, the artificial implant 100 may be manufactured by immersing a mold having a predetermined shape in a silicone solution and then performing drying and curing to form a silicone shell 110, applying or laminating a reinforcing material onto a desired site in the surface of the shell 110 and then performing drying and curing to form a reinforcing material layer, forming a silicone layer on the surface of the reinforcing material layer, repeatedly performing the formation of a reinforcing material layer and the formation of a silicone layer, and injecting a filler 120 into the silicone shell 110, but the present invention is not limited thereto.

Hereinafter, examples of the specification will be described in further detail. However, the following experimental results are obtained from only a few selected examples of the invention, and the scope and contents of the specification should not be interpreted as being reduced or limited by the few selected examples. The effects of each of the various embodiments of the specification, which are not explicitly set forth below, will be described in detail in relevant sections.

### Comparative Example

A shell sample consisting of silicone as a single material was manufactured.

### Example 1

A shell sample in which a polyester reinforcing material layer is interposed between silicone layers as shown in FIG. 4 was manufactured. In this case, silicone and a reinforcing material were used in a weight ratio of 9:1

### Example 2

A shell sample in which a polyester reinforcing material layer is interposed between silicone layers as shown in FIG. 4 was manufactured. In this case, silicone and a reinforcing material were used in a weight ratio of 8:2.

### Example 3

A shell sample in which a polyester reinforcing material layer is interposed between silicone layers as shown in FIG. 4 was manufactured. In this case, silicone and a reinforcing material were used in a weight ratio of 7:3.

### Example 4

A shell sample in which polyester reinforcing material layers are interposed between silicone layers as shown in FIG. 5 was manufactured. In this case, silicone and a reinforcing material were used in a weight ratio of 9:1.

### Example 5

A shell sample in which polyester reinforcing material layers are interposed between silicone layers as shown in FIG. 5 was manufactured. In this case, silicone and a reinforcing material were used in a weight ratio of 8:2.

### Example 6

A shell sample in which polyester reinforcing material layers are interposed between silicone layers as shown in FIG. 5 was manufactured. In this case, silicone and a reinforcing material were used in a weight ratio of 7:3.

All of the samples of Comparative Example and Examples 1 to 6 were manufactured to have the same thickness, and the weight ratios of the silicone and reinforcing material used in the samples are shown in the following Table 1.

**[Table 1]**

| Classification | Proportion of silicone (%) | Proportion of reinforcing material (%) |
|---|---|---|
| Comparative Example | 100 | 0 |
| Example 1 | 90 | 10 |
| Example 2 | 80 | 20 |
| Example 3 | 70 | 30 |
| Example 4 | 90 | 10 |
| Example 5 | 80 | 20 |
| Example 6 | 70 | 30 |

### Experimental Example 1

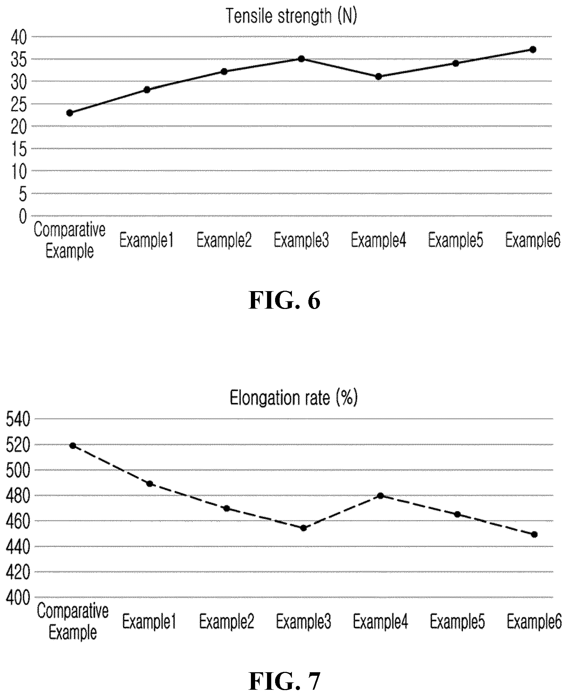

The tensile strength and elongation rate of each sample manufactured in Comparative Example and Examples 1 to 6 were measured and shown in the following Table 2 and FIGS. 6 and 7. Each sample was used after being cut into a dumbbell shape in accordance with the ISO 373 standard, and the tensile strength and elongation rate when the sample was broken were measured using a universal test machine (UTM).

**[Table 2]**

| Classification | Tensile strength (N) | Elongation rate (%) |
|---|---|---|
| Comparative Example | 23 | 520 |
| Example 1 | 28 | 490 |
| Example 2 | 32 | 470 |
| Example 3 | 35 | 455 |
| Example 4 | 31 | 480 |
| Example 5 | 34 | 465 |
| Example 6 | 37 | 450 |

The foregoing description of the specification is intended for illustration, and it will be understood by those skilled in the art to which the invention pertains that the invention can be easily modified in other specific forms without changing the technical spirit or essential features described in the specification. Therefore, it should be understood that the examples described above are only exemplary in all aspects and not limiting. For example, each of the constituents described as being one combined entity may be implemented separately, and similarly, constituents described as being separate entities may be implemented in a combined form.

It should be understood that the scope of the specification is defined by the following claims and that all changes or modifications derived from the meaning and scope of the claims and their equivalents are included in the scope of the specification.

### [List of Reference Numerals]

100: Artificial implant
101: Upper portion of artificial implant
102: Lower portion of artificial implant
103: Side portion of artificial implant
110: Silicone shell
111, 113, 115: Silicone layer
112, 114: Reinforcing material layer
120: Filler

## Claims

1. An artificial implant comprising:
a silicone shell; and
a filler filled in the shell,
wherein at least a portion of the shell is a rupture-resistant portion including two or more silicone layers and one or more reinforcing material layers interposed therebetween.

2. The artificial implant of claim 1, wherein a portion of the shell, which is not the rupture-resistant portion, has a thickness of 0.40 to 0.80 mm, and the rupture-resistant portion has a thickness of 0.40 to 1.20 mm.

3. The artificial implant of claim 1, wherein the shell further includes a barrier film layer.

4. The artificial implant of claim 1, wherein the rupture-resistant portion is provided in a side portion.

5. The artificial implant of claim 1, wherein the reinforcing material is at least one selected from polyester, polyurethane, polyvinyl chloride, nylon, polyethylene glycol, polyethylene oxide, polyhydroxyethylmethacrylate, poly(N-isopropylacrylamide), polycaprolactone, polylactic acid, polyglycolic acid, poly[(lactic-co-(glycolic acid))], polydioxanone, poly[(L-lactide)-co-(caprolactone)], poly(ester urethane), poly[(L-lactide)-co-(D-lactide)], poly[ethylene-co-(vinyl alcohol)], polyacrylic acid, polyvinyl alcohol, polyvinylpyrrolidone, gelatin, alginate, carrageenan, chitosan, hydroxyalkyl cellulose, alkyl cellulose, silicone, rubber, polyacrylacetate, titanium, titanium alloys, zirconia, alumina, nichrome, cochrome, and gold.

6. The artificial implant of claim 1, wherein the reinforcing material layer has at least one structure selected from a mesh, a woven fabric, a non-woven fabric, and a sheet.

7. The artificial implant of claim 1, wherein the rupture-resistant portion consists of silicone and a reinforcing material in a weight ratio of 55 to 95:5 to 45.

8. The artificial implant of claim 1, wherein the rupture-resistant portion has a tensile strength of 25 N or more.

9. The artificial implant of claim 1, wherein the rupture-resistant portion has an elongation rate of 300 to 500%.
